# EUROPEAN PATENT SPECIFICATION

(11) **EP 1 841 506 B1**
(45) Date of publication and mention of the grant of the patent: **25.04.2012**
(21) Application number: 06701661.8
(22) Date of filing: 18.01.2006
(51) Int. Cl.: A61Q 5/04

(54) **THE USE OF HIGH MOLECULAR WEIGHT CROSSLINKED, WATER-SOLUBLE CATIONIC POLYMERS IN HAIR CARE FORMULATIONS**
VERWENDUNG VON VERNETZTEN, WASSERLÖSLICHEN KATIONENPOLYMEREN MIT HOHEM MOLEKULARGEWICHT IN HAARPFLEGEFORMULIERUNGEN
UTILISATION DE POLYMERES CATIONIQUES RETICULES SOLUBLES DANS L'EAU A POIDS MOLECULAIRE ELEVE DANS DES FORMULATIONS DE SOIN CAPILLAIRE

(30) Priority: 27.01.2005 US 647668 P
(43) Date of publication of application: 10.10.2007
(73) Proprietor: BASF SE, 67056 Ludwigshafen (DE)
(72) Inventor: BAZEMORE, Emily Crisp, Winston Salem, NC 27107 (US); TSOTSOROS, Rhonda F., Hawthorne, NJ 07506 (US); SONG, Zhiqiang, Newtown, CT 06470 (US); MAO, Jianwen, New Milford, CT 06776 (US)
(86) International application number: PCT/EP2006/050268
(87) International publication number: WO 2006/079597

(56) References cited:
- WO-A-2004/018524
- US-A- 4 027 008
- US-A- 4 397 322
- US-A- 4 713 431
- US-A1- 2003 059 382
- US-B1- 6 682 725
- POLYMER PRODUCTS FROM ALDRICH, pages 50-51, XP002381000 Retrieved from the Internet: URL:http://www.sigmaaldrich.com/img/assets /3900/Viscosity.pdf> [retrieved on 2006-05-15]

## Description

This invention relates to a process for the preparation of hair conditioning formulations, comprising at least one high molecular weight, crosslinked, water-soluble cationic polymer as a conditioning agent. It further relates to non-therapeutic methods of treating hair, particularly methods of conditioning hair, comprising contacting hair with a hair care formulation comprising at least one high molecular weight, crosslinked, water-soluble cationic polymer as a conditioning agent.

Hair fibers are comprised of keratin, which is in turn composed of polypeptide chains bonded together by three types of bonds; cystine (or disulfide) bonds, hydrogen bonds and salt linkages. The relaxing process operates primarily on the cystine bonds. When the cystine bonds are exposed to an alkaline relaxing solution, they are transformed to lanthionine bonds. The high alkalinity hair straightener most commonly used contains sodium hydroxide, but may also contain other compounds such as thioglycolates, bisulfides, lithium, calcium and potassium hydroxide. Obviously there are many other compounds that are appropriate and applicable for this application based on similar principles of chemistry.

It is conceivable that the breakage of the cystine bonds results in further changes to the configuration of the hair fibers. Such changes could then be further utilized to create visual effects on the hair fibers as well as hair assemblies. For example, curly hair may become extended and straightened, i.e. a relaxing process. It is also entirely feasible that other visual effects such as a different degree of curling can be achieved with such a process.

As described in U.S. Patent Nos. 5,641,478 and 4,314,572, relaxing can be practically achieved by using either a multi-part straightener system or a single part straightener system.

Multi-part relaxer systems having two or more parts are known wherein the active hair relaxing ingredient, e.g. guanidine hydroxide, is generated in situ by combining two separate parts. For example part A of the multi-part system includes a relaxer cream that contains the active hydroxide metal ion, e.g. calcium hydroxide, and other ingredients such as conditioners. The liquid activator is in part B of the multi-part system. It consists of guanidine carbonate as stated in U.S. Patent No. 4,314,572. When parts A and B are mixed together, the calcium hydroxide and guanidine carbonate react to produce the active hair relaxing ingredient, product guanidine hydroxide.

Single part systems, which employ an alkali metal hydroxide such as sodium hydroxide, are also known.

A conventional relaxation process decreases the amount of curl in hair. Unfortunately such a process is also known to damage the hair. The relaxation process causes hair fibers to longitudinally split and break, leaving the hair coarse, brittle and unmanageable, as described in U.S. Patent No. 5,348,737. Therefore, it is desirable to apply a high performance conditioning system to re-condition and/or repair the damaged hair in order to achieve effects desired by consumers. To achieve such conditioning effects, the ingredients (conditioners) used have to be not only able to provide conditioning, but they also need to be substantive to the hair tresses to survive the washes and rinses following the relaxing process, and they must be compatible with the other ingredients used in the relaxers such as the alkalis. Another important point is that the conditioning polymer should not slow down the relaxing process and add to the irritation potential of the relaxer itself. The type and concentration of the conditioner chosen must be balanced within the relaxer formulation so that the irritation potential is not increased causing distress to the consumer using the relaxing process.

U.S. Patent No. 5,641,478 discloses hair strengthening compositions which comprise about 95 to about 99.5 wt % of a hair swelling component and about 0.5 to about 5 wt % of a cationic polymer, both based upon the total weight of the hair-strengthening composition. The preferred cationic polymer is the reaction product of a secondary amine and epihalohydrin in the presence of a small amount of ethylenediamine as crosslinker. Such polymers may be prepared as described, for instance, in Example 2 of U.S. Patent No. Re. 28,808.

The molecular weight of the preferred cationic polymer is in the range of about 400 to 600 x 10³. This cationic polymer in admixture with about 50-weight percent water was commercially available from Betz Laboratories, Trevose, PA, under the designation Betz Polymer 1195.

When Betz Polymer 1195 was replaced by other commercially available cationic polymers, such as Merquat 100, a linear poly diallyldimethyl ammonium chloride (polyDADMAC), or Polycare 133, a linear polymethylacrylamidopropyl trimonium chloride polymer, results that were inferior to the control (no cationic polymer) were obtained.

US 2003/059382 discloses a hair bodifier with a DADMAC copolymer of reduced specific viscosity (RSV) of 5.3.

US-A-4 027 008 discloses hair compositions with DADMAC polymer of viscosity 130 000 cP.

US-A-4 397 322 discloses a spa cleaner and a shampoo with a DADMAC polymer of high viscosity.

US -B1-6 682 725 discloses hair conditioners with cationic copolymers of vinyl Pyrrolidone. The K-values of these copolymers go beyond 100.

Surprisingly, it has been found that certain high molecular weight lightly crosslinked and water soluble cationic polymers as further described herein provide excellent conditioning effects to hair, especially in hair straightening and hair relaxing applications.

The high molecular weight lightly crosslinked and water soluble cationic polymer is a poly diallyldialkyl ammonium halide, for example a poly diallyldimethyl ammonium chloride (polyDADMAC) which is herein referred to as a HMW polyDADMAC.

Incorporation of from 0.05% to 20% by weight, for example from 0.1% to 15% by weight, preferably from 0.5% to 10.0% by weight of said high molecular weight slightly crosslinked and water soluble cationic polymer, which in one embodiment is a HMW, polyDADMAC, into a hair relaxer formulation, is found to be highly advantageous.

HMW polyDADMAC polymers exhibit excellent affinity to hair. It is also speculated that the lightly crosslinked structure of high molecular weight cationic polymers, for example HMW polyDADMAC polymers, allows easier removal to prevent excessive build-up of the polymeric materials on hair in subsequent shampooing processes. This may also be due to the improved film forming properties.

It is also speculated that, during a relaxing process, the swelling of the hair might allow the high molecular weight cationic polymer, for example a HMW polyDADMAC, to penetrate into the hair fiber cortex and form an elastic network in the cortex. If this takes place, it is conceivable that the high molecular weight cationic polymer would be locked inside the hair fiber after the relaxing solution is rinsed from the hair. This is believed to account for an increase the tensile strength of the hair fibers. It is also found that incorporating the high molecular weight cationic polymer, for example a HMW polyDADMAC polymer, into a relaxing or straightening formulation will diminish the harsh and raspy feel of the hair fibers that usually results from using conventional relaxing systems.

The use of high molecular weight cationic polymers, for example HMW polyDADMAC polymers, in relaxing or straightening systems also improves hair manageability and provides other benefits desired by consumers. For example the hair is more easily combed and styled, and has reduced breakage and friction and increased hair fiber smoothness.

Cationic polymers have been used extensively in home and personal care applications, water treatment, papermaking, mineral processing, petroleum recovery, fabrics, and pharmaceuticals. Among the most important and extensively used cationic polymers are the quaternary ammonium polymers of diallyldialkyl ammonium halide compounds. In fact, polymers of diallyldimethyl ammonium chloride are known in the personal care and cosmetic industry as polyquaternium 6, and are extensively used in skin and hair care applications.

Conventional, substantially linear quaternary ammonium polymers of diallyldialkyl ammonium chloride with a moderately high molecular weight may be prepared by the methods described in U.S. Patent No. 3,288,770. However the molecular weight of said polymers is not optimum for all applications. Therefore substantial research work has been carried out in order to prepare polyDADMACs with a higher molecular weight. Amongst the efforts, it is noted that WO 2004/018524 teaches that crosslinked and water-soluble polyDADMACs having a very high molecular weight, for example having a weight average molecular weight greater than 700,000 g/mole, preferably a weight average molecular weight greater than 850,000 g/mole, can be prepared by a post-polymerization crosslinking reaction.

With reference to the teachings in WO 2004/018524, it is noted that persulfate compounds, such as ammonium, sodium or potassium persulfate, are the most effective for crosslinking DADMAC polymers. The fact that persulfate compounds work most effectively may result from the fact that DADMAC polymers are cationic and persulfate is a difunctional anionic species before decomposition. The difunctional anionic persulfate may bring two DADMAC polymer chains together through ionic bonding before decomposing to form radicals for crosslinking through covalent bonding.

It is also noted that WO2004/018524 teaches that crosslinking of DADMAC polymers is hindered by residual monomer. Residual DADMAC monomer not only competes with the initiator for use but also causes the polymer to degrade. Polymers of DADMAC can be crosslinked by persulfate compounds only when the residual monomer is reduced to sufficiently low levels, which depend on the polymer concentration used for the post crosslinking.

Moreover, for polymers of DADMAC, feeding the same amount of the initiator over different lengths of time results in different viscosity increases or extents of crosslinking. Thus, varying the feed rate and the feed time of the initiator can easily control the extent of crosslinking.

It is also important to note that this reference provides a novel method to prepare a high-molecular-weight crosslinked water-soluble polymer of diallyldialkyl ammonium chloride, for example a crosslinked polymer of DADMAC, having a structure different from that of crosslinked polymers made by the addition of a conventional polyolefinic crosslinker. While the crosslinked polymers made using a polyolefinic crosslinker have the crosslinker as a bridge between two connected polymer chains, the crosslinked polymers of this reference do not contain such crosslinker bridges. Rather, they are believed to have shorter links between the polymer chains, with them being simply directly connected at some point on their backbones.

WO 2004/018524 also provides a process to obtain a higher molecular weight polymer by post-polymerization crosslinking at low polymer concentrations. The low polymer concentration used will not give a high in-process viscosity that can limit implementation in commercial production. The processes provided by this reference are believed to also enable the preparation of cationic polymers with controlled degree of crosslinking or branching and therefore a controlled molecular weight.

Surprisingly, it has now been found that the efficacy of the cationic polymers in hair care applications, particularly polyDADMACs, is dependent on their molecular weight and molecular architecture, i.e. whether they are linear, branched and/or crosslinked. Polymers prepared from the same monomers tend to give very different sensory properties in hair and skin care applications depending on the aforementioned parameters.

It has now been found, surprisingly, that water soluble and cationic polymers having a weight average molecular weight greater than 700,000 g/mole, for example a weight average molecular weight greater than 850,000 g/mole, and a branched and/or slightly crosslinked structure, especially HWM polyDADMACs such as those which can be prepared according to the teachings of the aforementioned reference, give superior performance to conventional polymers in hair care applications, particularly as conditioning agents for hair together with or after a beautification treatment such as relaxing, waving, perming, coloring, straightening or bleaching.

Thus, in one embodiment the present invention relates to a process for the preparation of a hair conditioner formulation which comprises at least one crosslinked, water-soluble cationic polymer having a weight average molecular weight of greater than 700,000 g/mole.

In one embodiment the water soluble and cationic polymer has a lightly crosslinked and/or branched molecular structure and comprises a high molecular weight polymer of diallyldimethylammonium chloride (DADMAC) which is obtainable by post-reaction crosslinking.

In one embodiment the present invention relates to a non-therapeutic method of treating hair, which comprises contacting hair with a hair care formulation comprising at least one crosslinked, water-soluble cationic polymer having a weight average molecular weight greater than 700,000 g/mole.
In one embodiment the method of treating hair is a hair conditioning method.

In one embodiment of the method the water soluble and cationic polymer has a lightly crosslinked and/or branched molecular structure and comprises a high molecular weight polymer of diallyldimethylammonium chloride (DADMAC) which is obtainable by post-reaction crosslinking.

The present invention is directed to the use of crosslinked, water-soluble cationic polymers having a weight average molecular weight greater than 700,000 g/mole, for example a weight average molecular weight greater than 850,000 g/mole, in hair conditioner formulations, that are used to treat the hair for relaxing, perming and waving.

In one embodiment the hair conditioning formulation comprises at least one water soluble and cationic polymer which is obtainable by a method comprising:
(a) polymerizing substantially all of diallyldialkylammonium halide by a reaction initiated by a free radical initiator to form a base cationic polymer solution;
(b) contacting the base cationic polymer solution with additional free radical initiator to cause multiple cationic polymer groups to form interconnecting bonds, so that said base cationic polymer solution forms an aqueous solution containing a multi-crosslinked cationic polymer having a higher molecular weight than the base cationic polymer, in particular having a weight average molecular weight greater than 700,000 g/mole, for example a weight average molecular weight greater than 850,000 g/mole.

The base polymers for crosslinking to prepare high molecular weight crosslinked water-soluble cationic polymers useful in the present invention can be produced by any known free radical polymerization method.

In one embodiment the cationic base polymers are those polymers made from polymerization of at least one diallyldialkylammonium halide compound, which may be represented by the following formula (1): 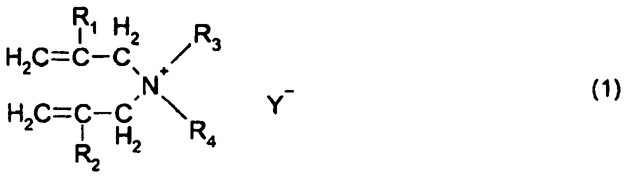 wherein
R₁ and R₂ are independently of one another hydrogen or C₁-C₄ alkyl;
R₃ and R₄ are, independently, hydrogen or alkyl, hydroxyalkyl, carboxyalkyl, carboxyamidalkyl or alkoxyalkyl groups having from 1 to 18 carbon atoms; and Y represents an anion, for example a halide. Examples of diallyldialkylammonium monomers include diallyldimethylammonium chloride (DADMAC), diallyldimethylammonium bromide, diallyldimethylammonium sulfate, diallyldimethylammonium phosphate, dimethyallyldimethylammonium chloride, diethylallyldimethylammonium chloride, diallyldi(beta-hydroxyethyl)ammonium chloride, diallyldi(beta-ethoxyethyl)ammonium chloride and diallyldiethylammonium chloride.

A preferred cationic monomer for the cationic base polymer is diallyldimethylammonium chloride. Thus, in one embodiment the water soluble and cationic polymer has a lightly crosslinked and/or branched molecular architecture and comprises a high molecular weight polymer of diallyldimethylammonium chloride (DADMAC) which is obtainable by post-reaction crosslinking.

The base polymers for crosslinking to prepare the high molecular weight crosslinked water-soluble cationic polymers useful in the present invention can also be any commercially available water-soluble cationic polymers, especially linear homopolymers or copolymers of diallyldialkylammonium halides. Examples of commercially available homopolymers or copolymers of diallyldialkylammonium halide include those sold under the trade names of Agefloc^{®}, Agequat^{®} and Salcare^{®} SC 30 by Ciba Specialty Chemicals Corporation.

Polymerization of at least one cationic monomer to form the cationic base polymer can be carried out by aqueous solution polymerization, water-in-oil inverse emulsion polymerization or dispersion polymerization using a suitable free radical initiator. Examples of suitable initiators include persulfates such as ammonium persulfate (APS); peroxides such as hydrogen peroxide, t-butyl hydroperoxide, and t-butyl peroxy pivalate, azo initiators such as 2,2'-azobis(2-amidinopropane) dihydrochloride, 4,4'-azobis-4-cyanovaleric acid and 2,2'-azobisisobutyronitrile; and redox initiator systems such as t-butyl hydroperoxide/Fe(II) and ammonium persulfate/bisulfite. Aqueous solution polymerization using ammonium persulfate (APS) is the preferred method for preparing the base cationic polymer of the preferred monomer, DADMAC.

The amount of the free radical initiator used in the polymerization process depends on total monomer concentration and the type of monomers used. It may range from about 0.2 to about 5.0 wt % of the total monomer charge to achieve more than 99% of total monomer conversion.

It is preferred to carry out the polymerization in the absence of oxygen. Oxygen can be removed from the reaction medium by applying vacuum with agitation or by purging with an inert gas such as nitrogen and argon. The polymerization can then be conducted under a blanket of the inert gas.

Diallylamine monomers such as DADMAC, although containing two unsaturated C=C double bonds, are well known to form linear polymers in the presence of a free radical initiator through cyclopolymerization. The linear polymers thus formed contain repeat units of 5 membered pyrrolidinium rings. It is desirable to make linear base polymers with as high a molecular weight as the free radical polymerization process can provide if a high molecular weight lightly crosslinked final product is desired. Reaction conditions such as monomer concentration, initiator concentration, reaction temperature and reaction time all combine to affect the rate of radical polymerization and molecular weight of the obtained base polymer. Those skilled in the art would be capable of selecting suitable reaction conditions to achieve a high molecular weight, for example a weight average molecular weight of greater than 100,000 g/mole.

The post-crosslinking technology disclosed in WO 2004/018524 can then be used to raise the molecular weight to an even higher value. By said process it is possible to obtain multi-crosslinked cationic polymers having a weight average molecular weight greater than 700,000 g/mole, for example a weight average molecular weight greater than 850,000 g/mole.

The intrinsic viscosity and the Huggins constant of said polymers are determined in 1 M NaCl aqueous solution at 30°C using standard procedures well known to one of ordinary skill in the art. In one embodiment the polymers prepared by the post-crosslinking technology disclosed in WO 2004/018524 have an intrinsic viscosity (in dUg) of 1.5 or more, for example 1.7 or more and especially 1.8 or more.

The cationic base polymer is chain extended or crosslinked by treating it with a suitable radical initiator in aqueous solution under agitation. A suitable radical initiator is a compound which can create radical sites on the cationic base polymer and help to overcome the positive electrostatic repulsion for combination of the cationic base polymeric radicals. Examples of suitable radical initiators are persulfate compounds such as potassium persulfate, sodium persulfate, ammonium persulfate, and the like. Other suitable radical initiators may include salts or derivatives of percarbonic acid (such as isopropyl percarbonate) and salts or derivatives of perphosphonic acid. The above-mentioned radical initiators may be used alone or in combination with various reducing agents to form redox initiator systems. Other polymerization initiators not mentioned above but known to those skilled in the art, may also be used for the crosslinking reaction under suitable reaction conditions. The most preferred radical initiators for crosslinking the cationic base polymers are ammonium persulfate, sodium persulfate and potassium persulfate in view of their crosslinking efficiency, water solubility and their decomposition temperature.

The radical initiator is used in an amount ranging from about 0.02 to about 50%, for example from about 0.5 to 10% and especially from about 1 to 5% by weight based on the cationic base polymer. The chain-extending or crosslinking reaction can be carried out in aqueous medium or in the same reaction medium (e.g., water-in-oil emulsion) as used for preparing the base polymer. The crosslinking reaction can be carried out in aqueous medium at a pH from about 1 to about 12, for example from 4 to 7, and at a temperature from about 20 to about 100°C, preferably from 70 to 100°C without using reducing agents. The solids concentration of the base polymer in the reaction medium prior to the crosslinking reaction can be from 1% to about 60% by weight, preferably from 10% to 30% for a solution base polymer, and preferably from 20 to 50% for an emulsion or dispersion base polymer.

The required initiator may be added all together in the reactor at the reaction temperature to crosslink the base polymer. However, addition of a large amount of the initiator may cause undesirable formation of water-insoluble gels. For better control of the molecular weight or viscosity advancement, the initiator can be added in small increments or at a modest continuous rate. The reaction is allowed to proceed after each incremental addition of the initiator (note: the increments can be made sufficiently small to be nearly a continuous addition) until the increase in the viscosity begins to level off. If the desired product viscosity has not yet been reached, another increment of initiator will be added. When the desired product viscosity is achieved, cooling to room temperature stops the reaction.

The preferred way to control the crosslinking reaction is by continuously feeding the initiator at a rate such that viscosity advancement of the reaction medium can be easily monitored. The efficiency of the initiator for crosslinking increases with decreasing feed rate of the initiator. Slow initiator feed rate gives high efficiency of the initiator for crosslinking and also provides easy control of viscosity or molecular weight advancement. The crosslinking reaction can be terminated once a desired viscosity or molecular weight is achieved by stopping the initiator feed and cooling the reaction.

The effect of the initiator after stopping the initiator feed is small if a slow initiator feed rate is used. The initiator can be fed to the aqueous solution of the base polymer at a rate of from 10% to 0.0005%, preferably from 0.2% to 0.001%, and most preferably from 0.05% to 0.002 % per minute by weight based on polymer solids. In this way multi-crosslinked cationic polymers having a weight average molecular weight greater than 700,000 g/mole, for example a weight average molecular weight greater than 850,000 g/mole, can be reproducibly obtained.

The persulfate dianion brings two cationic base polymer (H-P⁺) units together through ionic bonding. The homolytic decomposition of the persulfate produces two anionic sulfate radicals that abstract hydrogen atoms from the base polymer chains to create two polymer radicals. Crosslinking is effected only when two polymer radicals combine. The polymer radicals formed, if not finding each other for crosslinking, may undergo degradation through chain transfer or disproportionational termination. The persulfate dianions help to bring together for crosslinking two cationic polymer radicals, which would otherwise have difficulty approaching each other because of the cationic charge repulsion. Thus, persulfate initiators have a high efficiency for crosslinking cationic polymers. Other initiators, such as hydrogen peroxide, can create cationic polymer radicals. However, because of the difficulty of overcoming electronic repulsion forces for crosslinking, they tend to undergo degradation through chain transfer or termination. Moreover, radical initiators such as hydrogen peroxide may have a much higher tendency than persulfate to induce chain transfer degradation. Residual double bonds on the cationic base polymer may also play a role in crosslinking. The present inventors do not intend to be limited to any particular crosslinking mechanism.

In the above proposed crosslinking scheme, each persulfate molecule abstracts 2 hydrogen atoms to create two polymer radicals for crosslinking. The two abstracted hydrogen atoms are oxidized to two protons. Thus, the reaction pH will drift downward if no base is added to neutralize it. A decrease in pH is indeed observed with addition of persulfate initiator during the crosslinking reaction. The above-proposed mechanism is also supported by the experimental fact that a molar feed ratio of a base such as NaOH to ammonium persulfate of around 2.0 is optimal to achieve a high crosslinking efficiency and to keep the reaction pH relatively constant.

In order to keep the crosslinking reaction at a desired pH during the course of the initiator feed, a base may be added to keep the pH from drifting downward. Examples of suitable bases that can be used alone or in combination for pH control include NaOH, KOH, NH₄OH, Na₂CO₃, and the like. The preferred base for the pH control is aqueous NaOH.

In one embodiment the base can be added by continuous feeding at a fixed ratio with the initiator feed. The feed ratio of the base to the persulfate by moles can be from 0 to 8, preferably from 1 to 3, and the most preferably from 1.5 to 2.5. The base can also be added whenever the pH drops to below a desired value. As previously indicated, the crosslinking reaction can be carried out in an aqueous medium at a pH of from about 1 to about 12. However it is preferably carried out in an aqueous medium at a pH of from about 4 to 7.

In one embodiment pH controller is used control the pH of the crosslinking reaction. A base such as NaOH can be added to the reactor automatically by the pH controller whenever the reaction pH drifts below a desired value.

Polymers, for example of DADMAC, can be crosslinked by persulfate compounds only when residual monomer is reduced to sufficiently low levels. The maximum residual monomer level at which the crosslinking can occur depends on the polymer concentration used for the crosslinking reaction. Therefore, it is desirable that the cationic base polymer contains less than 10% of residual monomer, preferably less than 3%, and the most preferably less than 1% by weight of the base polymer solids. However, base polymers containing more than the desired amount of residual monomers can still be crosslinked by the methods disclosed in WO 2004/018524. In such cases, the radical initiator added in the crosslinking reaction is initially used for reduction of the residual monomer. Once the residual monomer is reduced to sufficiently low levels, the base polymer will begin crosslinking with the continued addition of initiator.

The chain-extension or crosslinking reaction is preferably carried out under agitation. Adequate agitation can help prevent formation of gel particles. Suitable agitation should not cause enough shear to result in significant polymer chain scission. In this way multi-crosslinked cationic polymers having a weight average molecular weight greater than 700,000 g/mole, for example a weight average molecular weight greater than 850,000 g/mole, which are substantially free of gel particles can be obtained.

Hair conditioner formulations according to the invention generally comprise from about 0.05 to 20% by weight, for example 0.1 to 15%, and especially 0.5 to 10%, based on the total weight of the preparation of a mixture of at least one crosslinked, water-soluble cationic polymer as described above, and a cosmetically acceptable carrier or diluent. While water is a cosmetically acceptable carrier or diluent and will normally also be present, the term "a cosmetically acceptable carrier or diluent" as used herein is meant to refer to a substance other than water.

Since the diluent only serves to dilute the polymer to allow uniform application of appropriately small quantities, any diluent that is physiologically acceptable for contact with the human body when used in a hair care composition may be used. In one embodiment, the polymer can be dissolved in water and/or an alcohol such as ethanol, isopropanol, propylene glycol, butylene glycol or glycerin, or mixtures thereof.

Suitable carriers for the hair conditioner formulations according to the invention are the conventional cosmetically acceptable materials used in such compositions. These may include inorganic or organic fillers, preservatives, UV filters and reflectors or other adjuvants and additives conventionally employed in hair care formulations.

The cationic polymer formulations as defined above are useful in methods for hair hair conditioning, such as thermal protection conditioners, hair-conditioning products, for example pretreatment products, hair rinses, deep conditioning treatments, intensive hair conditioning treatments. Depending on the specific hair treating application, the composition of this invention may be formulated by conventional means into pump spray, spritz, lotion, cream, gel, or mousse type compositions for easy application to hair, as well as other types of formulations capable of providing conditioning or any other cosmetic effects that would be familiar to those who are skilled in the art.

Thus one embodiment of the invention comprises a non-therapeutic method of treating hair, which comprises contacting hair with a hair conditioner formulation comprising at least one crosslinked, water-soluble cationic polymer having a weight average molecular weight greater than 700,000 g/mole, for example a weight average molecular weight greater than 850,000 g/mole as described above. The hair conditioner formulation may be applied in any suitable manner, such as by massaging the formulation throughout the hair by hand, by dipping the hair into the formulation, by brushing or combing the formulation throughout the hair or by spraying.

In one embodiment of the method the water soluble and cationic polymer has a lightly crosslinked and/or branched molecular architecture and comprises a high molecular weight polymer of diallyldimethylammonium chloride (DADMAC) which is obtainable by post-reaction crosslinking as described above.

In one embodiment of the method the cationic base polymers are those polymers made from polymerization of at least one diallyldialkylammonium halide compound, which may be represented by the formula (1): 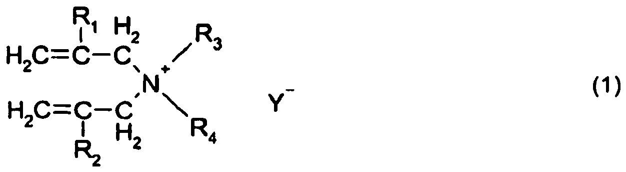 wherein
R₁ and R₂ are independently of one another hydrogen or C₁-C₄ alkyl;
R₃ and R₄ are, independently, hydrogen or alkyl, hydroxyalkyl, carboxyalkyl, carboxyamidalkyl or alkoxyalkyl groups having from 1 to 18 carbon atoms; and Y represents an anion, for example a halide. Examples of such diallydialkylammonium monomers include diallyldimethylammonium chloride (DADMAC), diallyldimethylammonium bromide, diallyldimethylammonium sulfate, diallyldimethylammonium phosphate, dimethyallyldimethylammonium chloride, diethylallyldimethylammonium chloride, diallyldi(beta-hydroxyethyl)ammonium chloride, diallyldi(beta-ethoxyethyl)ammonium chloride and diallyldiethylammonium chloride.

The term "hair" as used in the present invention includes treated and untreated human hair, animal hair, and any type of hair-like fiber that needs gloss, reduced fly-away and ease of combing. Treated hair includes hair that is chemically changed and/or damaged by lanthionization (relaxing), waving and/or dyeing (coloring), either temporarily, semi-permanently or permanently as known by those who are skilled in the art.

Creams are usually spreadable in the temperature range from room to skin temperature, whereas cream rinses, lotions or milks tend to be pourable.

Gels are semisolid systems in which the so-called gel former forms a three-dimensional network in which a liquid is immobilized. Clear to opaque hydrogels consist primarily of water, water-soluble substances and thickeners or gel formers.

In addition to the essential ingredients specified above, the formulation of this invention may comprise further ingredients (additives) which are conventional and/or beneficial.
Examples of such other ingredients (additives) include:
thickeners, emulsifiers and stabilizers, e.g., salts of various sorts, sodium alginate, gum arabic, polyoxyethylene, guar gum, hydroxypropyl guar gum, cellulose derivatives such as methylcellulose, methylhydroxypropylcellulose, hydroxypropylcellulose, polypropylhydroxyethylcellulose, starch and starch derivatives such as hydroxyethylamylose and starch amylose, and locust bean gum; thickeners; emulsifiers and/or stabilizers prepared by polymers and/or co-polymers of various acrylates, urethanes and/or other monomers as well as monomer combinations can also be used in such systems as long as they are either compatible, or can be made compatible by means of formulations and formulation aids, with the essential ingredients and that they provide the desirable attributes. It is also conceivable that polymers of specific architecture, such as block or other types of co-polymers and/or polyelectrolyte complex systems can also be used to thicken or otherwise stabilize the formulations;
perfumes;
hair root nutrients, such as such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e g. di-and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions of the invention is isoleucine and glucose. A particularly preferred amino acid nutrient is arginine;
polyols, such as such as glycerine and polypropylene glycol;
chelating agents, such as Na₂ EDTA, Na₃ EDTA;
foam boosters;
antifoam agents;
antioxidants;
antimicrobials;
sunscreens;
bactericides;
solvents, e.g., ethanol SDA40;
organic resins, e.g., polyquaternium 11;
emulsifiers, e.g., ceteareth 20, steareth 20, stearyl alcohol, and polysorbate 20;
emollient oils, e.g., dimethicone and cyclomethicone;
preservatives, e.g., methyl paraben, methylisothiazolinone;
opacifiers;
sequestering agents;
pH adjusting agents, e.g., citric acid;
dyes or any other substances that provide coloration;
substances of polymeric or non-polymeric nature that help protect or retain the hair colors;
specialty additives, such as re-fatting agents (e.g., isopropyl myristate and palmitate, cetyl alcohol, propylene glycol), pearlescent agents (e.g., ethylene glycol distearate), dandruff control agents (e.g., zinc pyrithione);
polysiloxanes, such as polydiorganosiloxanes, in particular polydimethylsiloxanes which have the CTFA designation dimethicone. Also suitable for use in compositions of the invention are polydimethyl siloxanes having hydroxyl end groups, which have the CTFA designation dimethiconol. It is preferred if the silicone oil also comprises a functionalized silicone. Suitable functionalized silicones include, for example, amino-, carboxy-, betaine-, quaternary ammonium-, carbohydrate-, hydroxy-and alkoxy-substituted silicones. Preferably, the functionalized silicone contains multiple substitutions. For the avoidance of doubt, as regards hydroxyl-substituted silicones, a polydimethylsiloxane merely having hydroxyl end groups (which have the CTFA designation dimethiconol) is not considered a functionalized silicone within the present invention. However, a polydimethylsiloxane having hydroxyl substitutions along the polymer chain is considered a functionalized silicone. Suitable amino functionalized silicones are described in EP 455,185 (Helene Curtis) and include trimethylsilylamodimethicone as depicted below, and are sufficiently water insoluble so as to be useful in compositions of the invention: Si(CH₃)₃-O-[Si(CH₃)₂-O-]ₓ-[Si(CH₃)(R-NH-CH₂CH₂NH₂)O-]_{y}-Si(CH₃)₃ wherein x + y is a number from about 50 to about 500, and the weight percent amine functionality is in the range of from about 0.03% to about 8% by weight of the molecule, and wherein R is an alkylene group having from 2 to 5 carbon atoms. As expressed here, the weight percent amine functionality is measured by titrating a sample of the amino-functionalized silicone against alcoholic hydrochloric acid to the bromocresol green end point. The weight percent amine is calculated using a molecular weight of 45 (corresponding to CH₃-CH₂NH₂). Suitably, the weight percent amine functionality measured and calculated in this way is in the range from 0.03% to 8%, preferably from 0. 5% to 4%. An example of a commercially available amino-functionalized silicone useful in the silicone component of the composition of the invention is DC-8566 available from Dow Coming (INCl name: dimethyl, methyl (aminoethylaminoisobutyl) siloxane). This has a weight percent amine functionality of about 1.4%. Examples of suitable amino functional silicones include: polysiloxanes having the CTFA designation" Aminopropyl Dimethicone". Specific examples of amino functional silicones suitable for use in the invention are the aminosilicone oils DG8220, DC-8166, DC-8466, and DC-8950-114 (all ex Dow Corning), GE 1149-75, (ex General Electric Silicones), and TINOCARE^{®} Si A1 (ex Ciba Specialty Chemicals). Suitable quaternary silicone polymers are described in EP-A-0 530 974. A preferred quaternary silicone polymer is K3474, ex Goldschmidt. Another preferred functional silicone for use as a component in the hydrophobic conditioning oil is an alkoxy-substituted silicone. Such molecules are known as silicone copolyols and have one or more polyethylene oxide or polypropylene oxide groups bonded to the silicone polymer backbone, optionally through an alkyl linking group. A non-limiting example of a type of silicone copolyol useful in compositions of the invention has a molecular structure according to the formula: Si(CH₃)₃[O-Si(CH₃)(A)]ₚ- [O-Si (CH₃)(B)]_{q}-O-Si(CH₃)₃. In this formula, A is an alkylene chain with from 1 to 22 carbon atoms, preferably 4 to 18, more preferably 10 to 16. B is a group with the structure: -(R)-(EO)ᵣ(PO)S-OH wherein R is a linking group, preferably an alkylene group with 1 to 3 carbon atoms. Preferably R is -(CH₂)₂-. The mean values of r and s are 5 or more, preferably 10 or more, more preferably 15 or more. It is preferred if the mean values of r and s are 100 or less. In the formula, the value of p is suitably 10 or more, preferably 20 or more, more preferably 50 or more and most preferably 100 or more. The value of q is suitably from 1 to 20 wherein the ratio p/q is preferably 10 or more, more preferably 20 or more. The value of p + q is a number from 11 to 500, preferably from 50 to 300.

Suitable silicone copolyols have an HLB of 10 or less, preferably 7 or less, more preferably 4 or less. A suitable silicone copolyol material is DC5200, known as Lauryl PEG/PPG-18/18 methicone (INCI name), available from Dow Corning.
It is preferred to use a combination of functional and non- functional silicones as the hydrophobic silicone conditioning oil. Preferably the silicones are blended into common droplets prior to incorporation into compositions according to the invention.

The viscosity of the hydrophobic silicone conditioning oil, measured in isolation from the rest of the composition (i.e. not the viscosity of any pre-formed emulsion, but of the hydrophobic conditioning oil itself) is typically from 350 to 200,000,000 mm²sec⁻¹ at 25°C. Preferably the viscosity is at least 5,000 mm²sec⁻¹ at 25°C, more preferably at least 10,000 mm²sec⁻¹. Preferably the viscosity does not exceed 20,000,000 mm²sec⁻¹, more preferably 10,000,000 mm²sec⁻¹, most preferably 5,000,000 mm²sec⁻¹;
conventional hair conditioning agents such as waxes, oils, stearalkonium chloride, dicetyldimonium chloride, stearamidopropyl dimethylamine, and other quaternary organic compounds; and
additives that reduce static electricity build-up and fly-away. One embodiment of such an additive is a quaternary amine.

Each of these ingredients will be present in an amount effective to accomplish its purpose. Generally these optional ingredients are included individually at a level of up to 5% by weight of the total hair treatment formulation.

In one embodiment of the inventive method, the hair treating formulation of this invention is a conditioning product which is applied to hair after shampooing. The hair is typically rinsed in running water after treatment with the conditioning formulation. Conditioners facilitate combing out hair and impart softness and suppleness to the hair. Conditioning compositions may also contain other components such as thickeners and auxiliary conditioning compounds. Auxiliary conditioning agents may be used to provide further improved conditioning benefits such as antistatic characteristics. Auxiliary conditioning agents useful in the composition of this invention include organic cationic compounds and polymers such as stearyldimethylbenzylammonium chloride or bromide, lauryl-trimethylammonium chloride or bromide, dodecyldimethylhydroxyethylammonium chloride or bromide, dimethyldistearylammonium chloride or bromide and dimethyldilaurylammonium chloride or bromide, quaternary nitrogen derivatives of cellulose ethers, and homopolymers and copolymers of diallyldimethylammonium chloride such as the SALCARE^{®} range of hair conditioning polymers available from Ciba Specialty Chemicals Corporation, Tarrytown, NY, homopolymers or copolymers derived from acrylic acid or methacrylic acid containing cationic nitrogen functional groups attached to the polymer via ester or amide linkages, copolymers of vinylpyrrolidone and acrylic acid esters with quaternary nitrogen functionality and other quaternary ammonium compounds which are known for use in hair conditioning formulations. They are used in conventional amounts to attain the desired effects.

When the hair treating composition of this invention is a conditioning product for application to hair after shampooing, it contains, in addition to about 0.1 to 10 parts by weight of the above-described cationic polymer and the diluent, from 1 up to about 4 parts of refatting agents such as fatty alcohols, for example cetyl or stearyl alcohol and waxes or lanolin derivatives. Additionally it may contain from 0.2 up to 3.0 parts of secondary conditioning agents such as natural oils and silicones, from 0 up to 6 parts of emulsifiers such as nonionic surfactants and liquid dispersion polymers such as the SALCARE^{®} SC92, SC95, SC96 polymers available from Ciba Specialty Chemicals Corporation, Tarrytown, NY, and conventional amounts of other adjuvants such as proteins, polymeric resins and gums, preservatives, pH and viscosity adjusters, colorants and perfumes, to name just a few, each by weight of the total composition.

In one embodiment of the inventive method, the hair treating formulation of this invention is a leave-in conditioner. A leave-in conditioner additionally advantageously contains from 0.5 up to 7 parts of primary conditioning agents, for example cationic surfactants like dicetyldimonium chloride and cetrimonium chloride.

Aerosol mousse formulations typically contain 8 to 15 parts by weight of gaseous propellants, and gel formulations typically contain 0.25 to 1 parts by weight of a gelling agent/thickener.

In one embodiment the present invention also includes a method of treating hair, which comprises applying to the surface of the hair an effective amount of a hair conditioning formulation comprising at least one crosslinked, water-soluble cationic polymer having a weight average molecular weight greater than 700,000 g/mole.

After the composition is applied, the hair may or may not be rinsed, depending on whether the composition applied is a rinsable or non-rinsable composition.

Generally, the amount of hair treating formulation that is applied is that amount which is effective to thoroughly coat the hair. The amount required will vary with the quantity and type of hair of each individual. Appropriate amounts for any individual's hair are readily determined by one or several trial applications. The length of time in which the conditioner should be left on the hair will also vary according to hair type. Generally, if the hair treating composition is a rinsable conditioner, it is left on the hair for a period of from at least about 30 seconds to about 5 minutes.

In one embodiment of the method, the hair conditioner formulation is applied before, during or after, preferably after a straightening (lanthionization) step.

In one embodiment of the method a hair conditioner formulation comprising
a) 0.05 to 10 wt-%, for example 0.1 to 8 wt-% and especially 0.1 to 5 wt-%, based on the total weight of the formulation, of at least one crosslinked, water-soluble cationic polymer as described above,
b) 0.5 to 5 wt-%, for example 1 to 4 wt-%, based on the total weight of the formulation, of at least one long chain fatty alcohol,
c) at least one skin compatible acid in an amount sufficient to obtain a pH between 2.5 to 5.5, for example 3-5 and especially around 3.5,
d) 0 to 5 wt-%, based on the total weight of the formulation, of at least one further additive, and
e) water up to 100 wt-% is applied.

The long chain fatty alcohol may contain 12 to 22 carbon atoms, preferably 16-18 carbon atoms.

The acid can for example be citric, lactic, tartaric, adipic or phosphoric acid or their salts.

The composition can also contain a thickener, for example a cellulose-based thickener such as ethyl hydroxyethyl cellulose.

Another optional ingredient is a quaternary ammonium surfactant, such as a mono- di-or trialkyl quat and/or a mono- di- or triacyl ester quat. The quaternary compounds may also be ethoxylated.

Other ingredients that may be added are emulsifiers; oils such as silicone oils, triglycerides or mineral oil; dyes, humectants, polyols, vitamins and hydrophobic esters containing either a long chain fatty acid or a long chain fatty alcohol.

In the examples all parts given are by weight unless otherwise indicated.

The symbols below are used in the following examples:
APS = ammonium persulfate
BV = Brookfield viscosity, cps
DAA = diallylamine
GPC = gel permeation chromatography
HC = Huggins constant
IV = intrinsic viscosity (measured in 1 M NaCl solution), dUg at 30°C.
Mw = weight average molecular weight (by GPC using PEO standard), g/mole
Mn = number average molecular weight (by GPC using PEO standard), g/mole
NaPS = sodium persulfate
PS = polymer solids, wt%
RM = residual monomer (of DADMAC), wt%
MBS = sodium metabisufite

### Example 1

A 20% by weight aqueous solution of a linear polyDADMAC which is commercially available from Ciba Specialty Chemicals, is used as the cationic base polymer for chain extension in this example. The cationic base polymer has the properties shown in Table 1. The intrinsic viscosity and the Huggins constant are determined in 1 M NaCl aqueous solution at 30°C using standard procedures well known to one of ordinary skill in the art. The weight average molecular weight, Mw, and number average molecular weight, Mn, are determined by GPC. The Mw/Mn ratio or polydispersity index is an indication of molecular weight distribution, with high value indicating a broad distribution.

A 1-liter reactor fitted with a mechanical agitator, addition funnel and condenser is charged with 964.00 grams of the 20% aqueous solution of the base polymer. The reactor content is adjusted with NaOH solution to a pH of 6.9 and then heated to 100°C with agitation and a nitrogen purge. At 100°C, 18.2 g of 10% APS solution is fed to the reactor over 160 minutes and then another 19.0 g of 10% APS over 157 minutes. During the APS feeds, a 25% NaOH solution is co-fed to the reactor at a rate to give a NaOH/APS feed molar ratio of 2.0. Total APS used is 1.9 % based on polymer solids. After the APS/NaOH co-feeds, the reactor content is held at 100°C for 10 minutes and then cooled to room temperature. A product free from water-insoluble gel is obtained with the properties shown in Table 1.

**Table 1. Properties of the cationic base polymer and its chain extended product of Example 1:**

| Properties at 25°C | RM, % | PS % | pH | BV, cps | Mw x10⁻³ | Mw/Mn | IV, dL/g | HC |
|---|---|---|---|---|---|---|---|---|
| Base polymer used | < 0.1 | 20.6 | 5.4 | 3080 | 620 | 6.30 | 1.40 | 0.36 |
| chain extended product | < 0.1 | 19.9 | 6.8 | 8040 | 966 | 10.2 | 1.86 | 0.53 |

### Example 2

A 20% by weight aqueous solution of a linear polyDADMAC which is commercially available from Ciba Specialty Chemicals, is used as the cationic base polymer for chain extension. The cationic base polymer used has properties shown in Table 2.

A 1-liter reactor fitted with a mechanical agitator, addition funnel and condenser is charged with 964.00 grams of the 20% aqueous solution of the base polymer. The reactor content is heated to 100°C with agitation and a nitrogen purge. At 100°C, 26.5 g of 10% APS solution is fed to the reactor over 170 minutes and then another 11.0 g of 10% APS over 90 minutes. During the APS feeds, the reaction pH is controlled at about 5.0 using a Chemcadet pH controller with a 25% NaOH solution. Total APS used is 1.9% based on polymer solids. After the APS feed, the reactor content is held at 100°C for 20 minutes and then cooled to room temperature. A gel-free clear polymer solution product is obtained with the properties shown in Table 2.

**Table 2. Properties of the cationic base polymer and its chain extended product of Example 2:**

| Properties at 25°C | RM, % | PS% | pH | BV, cps | Mw x10⁻³ | Mw/Mn | IV, dL/g | HC |
|---|---|---|---|---|---|---|---|---|
| Base polymer used | < 0.1 | 20.6 | 5.4 | 3080 | 620 | 6.30 | 1.40 | 0.36 |
| chain extended product | < 0.1 | 19.9 | 5.9 | 8550 | 929 | 10.9 | 1.83 | 0.42 |

### Example 3

### Evaluation of conditioning polymers

Various conditioning polymers were evaluated at low pH (2) and high pH (13). They were visually evaluated for precipitation and odor. The polymers were re-evaluated after one week in an oven at 45°C.

### Procedure

1% active solutions were prepared using the testing conditioning agents:

The pH of each solution was measured and then lowered to 2 using 10% phosphoric acid. The samples were then evaluated for precipitation and odor. Another 1% active solution was prepared and the pH was measured and then raised to13 using NaOH pellets. The samples were then evaluated for precipitation and odor.

Samples were stored at 25°C and 45°C and evaluated at one-week intervals.

### Example 4

### Preparation of Relaxer Base, parts

| | | |
|---|---|---|
| Water | | 57.0 |
| Mineral Oil | Drakeol 9Lt | 15.0 |
| Cetearyl Alcohol (and) Dicetyl Phosphate (and) Ceteth-10 Phosphate | Crodafos CES | 7.5 |
| Petrolatum | Snow White Pet | 5.5 |
| Calcium Hydroxide | Calcium Hydroxide | 5.0 |
| Propylene Glycol | Propylene Glycol | 3.0 |
| Behenyl Trimonium Methylsulfate | Behenyl TMS | 2.0 |
| Steareth 10 | Volpo S-10 | 2.0 |
| Cetyl Alcohol | Lanette-16 | 1.0 |
| Steareth 20 | Volpo S 20 | 0.50 |
| Polymer of Ex. 1 | | 1.5 |

| | | |
|---|---|---|
| PH: 11.50-12.50 Wt % Actives 4.8-5.0% | | |

**Activator, parts**

| | | |
|---|---|---|
| Water | Water | 74.80 |
| Xanthum Gum | Xanthum Gum | 0.20 |
| Guanidine Carbonate | Guanidine Carbonate | 25.0 |
| Red No. 33 | D&C Red No. 33 | Trace |

| | | |
|---|---|---|
| PH: 3.0-4.0 Wt. % Active: 24.5-25.0 | | |

**Relaxer Base w and w/o lithium hydroxide, parts**

| | | |
|---|---|---|
| Water | | 40.15 |
| Mineral Oil | Drakeol 9Lt | 15.0 |
| Cetearyl Alcohol (and) Dicetyl Phosphate (and) Ceteth-10 Phosphate | Crodafos CES | 7.0 |
| Petrolatum | Snow White Pet | 21.0 |
| Calcium Hydroxide | Calcium Hydroxide | 5.0 |
| Propylene Glycol | Propylene Glycol | 3.0 |
| Lithium Hydroxide | Lithium Hydroxide | 2.1 |
| Steareth 10 | Volpo S-10 | 1.75 |
| Cetyl Alcohol | Lanette-16 | 1.0 |
| Steareth 2 | Volpo S 2 | 0.50 |
| Stearyl Alcohol | Lanette- 18 | 2.0 |
| Polymer of Ex. 1 | | 1.5 |

**Activator, parts**

| | | |
|---|---|---|
| Water | Water | 74.80 |
| Xanthum Gum | Xanthum Gum | 0.20 |
| Guanidine Carbonate | Guanidine Carbonate | 25.0 |
| Red No. 33 | D&C Red No. 33 | Trace |

| | | |
|---|---|---|
| PH: 3.0-4.0 % Active: 24.5-25.0 | | |

### Example 5

### Curl Rearranger Neutralizer

(Neutralizer designed to neutralize the hair after chemical straightener treatment. It is applied to hair after perming to bring the hair back to its isoelectric pH).

**Composition**

| | Trade name | INCI-Name | Supplier | % w/w (as supplied) |
|---|---|---|---|---|
| Part A | Deionized water | | | qs |
| | Propylene Glycol | Propylene Glycol | | 2.0 |
| | Polymer of Ex. 1 | Polyquaternium-6 | | 1.50 |
| | | | | |
| Part B | Urea | Urea | | 6.0 |
| | Sodium Bromate | Sodium Bromate | | 10.50 |
| | | | | |
| Part C | DL-Panthenol | Panthenol | | 0.10 |
| | Fragrance | | | 0.20 |
| | Puricolor Blue ABL9 1 % sol. | FD&C Blue #1 | | 0.10 |

### Technical Data

| | |
|---|---|
| pH value | 6.5-7.5 |
| Appearance | Water thin liquid |
| Viscosity (Brookfield RVT/ Spindle 6/20 rpm) | 50-120 cps |

### Manufacturing instructions

In a large vessel add Part A with good agitation.
Add Part B and continue mixing until free of lumps.
Add Part C singularly with mixing in between until homogeneous

### Example 6

Waving or straightening formulation (can be used for both)
Curl Rearranger (hair straightener designed to relax the natural curl or curl naturally straight hair).

**Composition**

| | Trade name | INCI-Name | Supplier | % w/w (as supplied) |
|---|---|---|---|---|
| Part A | Deionized water | | | qs |
| | | | | |
| Part B | Promulgen D | Cetearyl Alcohol (and) | | 8.0 |
| | | Ceteareth-20 | | |
| | Lanette 18 NF | Stearyl Alcohol | | 3.0 |
| | | | | |
| Part C | Volpo G-31 | Glycereth-31 | | 2.0 |
| | Polymer of Ex. 1 | Polyquaternium-6 | | 1.50 |
| | | | | |
| Part D | Deionized water | | | 3.0 |
| | Ammonium Thioglycolate | Ammonium | | 11.65 |
| | 60% | Thioglycolate | | |
| | Ammonium Hydroxide | Ammonium hydroxide | | 3.85 |
| | 28% | | | |
| | | | | |
| Part E | TEA 99% | | | PH 9.5-10.50 |
| | Fragrance | | | 0.10 |

### Technical Data

| | |
|---|---|
| pH value | 9.5-10.50 |
| Appearance | Slightly Viscous, opaque lotion/cream |
| Viscosity (Brookfield RVT/ Spindle 6/20 rpm) | 3500-6000 cps |

### Manufacturing instructions:

In large vessel add Part A with good agitation. Begin heating to 80°C.
At 80°C, add Part B and continue mixing for 10 minutes.
Turn off heat. Cool to 60°C and add Part C singularly with mixing in between until homogeneous.
Premix Part D and add to vessel at 40-45°C. Mix until homogenous.
Add Part E if needed to adjust pH.

### Example 7

### Evaluation of a relaxer base

The polymer of Example 1 and comparative polymers A, B, C and D were incorporated into relaxer bases at 2.0% w/w and were applied to African hair tresses relaxed 1X and the reduction in combing force was measured by Diastron analysis. The data resulting from this operation consists of a graph showing the load in (in grams) opposing (or generated by) combing as a function of the position of the comb along the length of the swatch. This graph is called a 'Combing Curve'. The data are reported as (gmf).

The samples were also applied to African hair tresses relaxed 1X and evaluated for sensory testing. Parameters evaluated were: Wet: feel, removal of tangles, conditioned feel, comb drag, residue left on comb; Dry: comb drag after blow dry, feel on hands; curled for 30 seconds; snap after curling, body after curling, comb drag after curling. Results were averaged and polymers were given an overall sensory result based on a forced ranking by an expert panel.
Results:

| Product | Diastron Results Average Combing Force (gmf) | Overall Sensory Results: Positive results from lowest to highest |
|---|---|---|
| Polymer of Ex. 1 | 13.32 | 2 |
| Polymer A | 14.11 | 2 |
| Polymer B | 15.71 | 3 |
| Polymer C | 12.71 | 3 |
| Polymer D | 30.83 | 4 |

| | | |
|---|---|---|
| 1 = soft 5 = harsh Polymer A is a commercial high molecular weight polyDADMAC in bead form, intrinsic viscosity (IV) about 1.1; Polymer B is an aqueous solution of a commercial crosslinked polyepiamine with 50% solids and a viscosity of about 6000 cps, IV about 0.5; Polymer C is an aqueous solution of a commercial high molecular weight polyDADMAC, IV about 1.0, and Polymer D is an aqueous solution of a commercial medium molecular weight polyDADMAC, IV about 0.5. | | |

Diastron Analysis data showed that Polymer C and the HMW polyDADMAC of example 1 had similar combing reduction in which it took less force to comb the hair tress. The Overall Sensory Testing demonstrated that the HMW polyDADMAC was similar to Polymer A with respect to conditioning and softness and better than Polymers C and D.

## Claims

1. A process for the preparation of a hair conditioner formulation comprising at least one crosslinked, water-soluble cationic polymer having a weight average molecular weight greater than 700,000 g/mol, wherein the crosslinked, water-soluble cationic polymer is obtainable by a method comprising:
(a) polymerizing substantially all of diallyldialkylammonium halide compound by a reaction initiated by a free radical initiator to form a base cationic polymer solution;
(b) contacting the base cationic polymer solution with additional free radical initiator to cause multiple cationic polymer groups to form interconnecting bonds, so that said base cationic polymer solution forms an aqueous solution containing a multi-crosslinked cationic polymer.

2. A process according to claim 1, wherein the prepared formulation comprises from 0.05 to 20% by weight of at least one crosslinked, water-soluble cationic polymer having a weight average molecular weight of greater than 700,000 g/mole according to claim 1 and at least one cosmetically acceptable carrier or diluent which is other than or in addition to water.

3. A process according to claim 1, wherein at least one monomeric diallyldialkylammonium halide compound is represented by the formula (1): 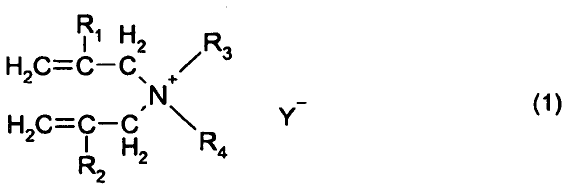 wherein
R₁ and R₂ are independently of one another hydrogen or C₁-C₄ alkyl;
R₃ and R₄ are, independently, hydrogen or alkyl, hydroxyalkyl, carboxyalkyl, carboxyamidalkyl or alkoxyalkyl groups having from 1 to 18 carbon atoms; and
Y⁻ represents an anion.

4. A process according to claim 3, wherein at least one monomeric diallyldialkylammonium halide compound is diallyldimethylammonium chloride, diallyldimethylammonium bromide, diallyldimethylammonium sulfate, diallyldimethylammonium phosphate, dimethyallyldimethylammonium chloride, diethylallyldimethylammonium chloride, diallyldi(beta-hydroxyethyl)ammonium chloride, diallyldi(beta-ethoxyethyl)ammonium chloride or diallyldiethylammonium chloride.

5. A process according to claim 1, wherein the crosslinked, water-soluble cationic polymer has a lightly crosslinked and/or branched molecular architecture and comprises a high molecular weight polymer of diallyldimethylammonium chloride which is obtainable by post-reaction crosslinking.

6. A non-therapeutic method of treating hair, which comprises contacting hair with a hair conditioner formulation, comprising
a) 0.05 to 10 wt-% based on the total weight of the formulation, of at least one crosslinked, water-soluble cationic polymer as described in claim 1,
b) 0.5 to 5 wt-% based on the total weight of the formulation, of at least one long chain fatty alcohol,
c) at least one skin compatible acid in an amount sufficient to obtain a pH between 2.5 to 5.5,
d) 0 to 5 wt-%, based on the total weight of the formulation, of at least one further additive, and
e) water up to 100 wt-%.

7. A method according to claim 6, which comprises contacting hair with a hair conditioner formulation in the form of rinsing products to be applied after shampooing, before or after tinting, dyeing, or bleaching, and before or after permanent waving or straightening; products for setting or brushing: conditioning compositions; restoring compositions; or compositions for permanent-waved hair.

8. A method according to claim 7, which comprises contacting hair with a hair conditioner formulation, which is applied before, during or after a straightening step.

## Patentansprüche

1. Verfahren zur Herstellung einer Haarkonditioniermittelformulierung, umfassend mindestens ein vernetztes, wasserlösliches kationisches Polymer mit einem gewichtsmittleren Molekulargewicht von mehr als 700.000 g/mol, wobei das vernetzte, wasserlösliche kationische Polymer nach einem Verfahren erhältlich ist, bei dem man:
(a) eine Diallyldialkylammoniumhalogenidverbindung durch eine durch einen radikalischen Initiator initiierte Reaktion weitgehend vollständig polymerisiert, wobei man eine Lösung von kationischem Basispolymer erhält;
(b) die Lösung von kationischem Basispolymer mit zusätzlichem radikalischem Initiator in Berührung bringt, um mehrere kationische Polymergruppen zur Bildung von verknüpfenden Bindungen zu veranlassen, so dass die Lösung von kationischem Basispolymer eine wässrige Lösung bildet, die ein mehrfach vernetztes kationisches Polymer enthält.

2. Verfahren nach Anspruch 1, bei dem die hergestellte Formulierung 0,05 bis 20 Gew.-% mindestens eines vernetzten, wasserlöslichen kationischen Polymers mit einem gewichtsmittleren Molekulargewicht von mehr als 700.000 g/mol nach Anspruch 1 und mindestens einen kosmetisch unbedenklichen Träger oder mindestens ein kosmetisch unbedenkliches Verdünnungsmittel, das von Wasser verschieden ist oder neben Wasser vorliegt, umfasst.

3. Verfahren nach Anspruch 1, bei dem mindestens eine monomere Diallyldialkylammoniumhalogenidverbindung durch die Formel (1) wiedergegeben wird: 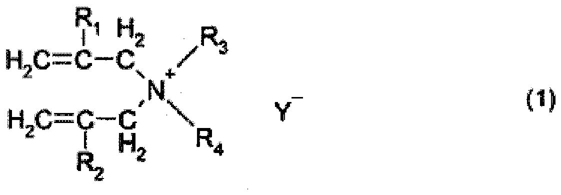 worin
R₁ und R₂ unabhängig voneinander für Wasserstoff oder C₁-C₄-Alkyl stehen;
R₃ und R₄ unabhängig voneinander für Wasserstoff oder Akyl-, Hydroxyalkyl-, Carboxyalkyl-, Carboxyamidalkyl- oder Alkoxyalkylgruppen mit 1 bis 18 Kohlenstoffatomen stehen und
Y⁻ für ein Anion steht.

4. Verfahren nach Anspruch 3, bei dem es sich bei mindestens einer monomeren Diallyldialkylammoniumhalogenidverbindung um Diallyldimethylammoniumchlorid, Diallyldimethylammoniumbromid, Diallyldimethylammoniumsulfat, Diallyldimethylammoniumphosphat, Dimethylallyldimethylammoniumchlorid, Diethylallyldimethylammoniumchlorid, Diallyldi-(beta-hydroxyethyl)ammoniumchlorid, Diallyldi-(beta-ethoxyethyl)ammoniumchlorid oder Diallyldiethylammoniumchlorid handelt.

5. Verfahren nach Anspruch 1, bei dem das vernetzte, wasserlösliche kationische Polymer eine leicht vernetzte und/oder verzweigte molekulare Architektur aufweist und ein hochmolekulares Polymer von Diallyldimethylammoniumchlorid, das durch Nachvernetzung erhältlich ist, umfasst.

6. Nichttherapeutisches Verfahren zur Behandlung von Haar, bei dem man Haar mit einer Haarkonditioniermittelformulierung, umfassend
a) 0,05 bis 10 Gew.-%, bezogen auf das Gesamtgewicht der Formulierung, mindestens eines vernetzten, wasserlöslichen kationischen Polymers gemäß Anspruch 1,
b) 0,05 bis 5 Gew.-%, bezogen auf das Gesamtgewicht der Formulierung, mindestens eines langkettigen Fettalkohols,
c) mindestens eine hautverträgliche Säure in einer zum Erhalt eines pH-Werts zwischen 2,5 und 5,5 ausreichenden Menge,
d) 0 bis 5 Gew.-%, bezogen auf das Gesamtgewicht der Formulierung, mindestens eines weiteren Additivs, und
e) Wasser bis 100 Gew.-%,
in Berührung bringt.

7. Verfahren nach Anspruch 6, bei dem man Haar mit einer Haarkonditioniermittelformulierung in Form von nach dem Shamponieren, vor oder nach dem Tönen, Färben oder Bleichen und vor oder nach Dauerwellung oder Entkräuselung aufzubringenden Spülprodukten; Produkten zum Festigen oder Bürsten; Konditionierzusammensetzungen; Wiederherstellungszusammensetzungen oder Zusammensetzungen für dauergewelltes Haar in Berührung bringt.

8. Verfahren nach Anspruch 7, bei dem man Haar mit einer Haarkonditioniermittelformulierung, die vor, während oder nach einem Entkräuselungsschritt aufgebracht wird, in Berührung bringt.

## Revendications

1. Procédé d'élaboration d'une formule de conditionnement capillaire comprenant au moins un polymère cationique hydrosoluble réticulé de masse moléculaire moyenne en poids supérieure à 700 000 g/mol, où le polymère cationique hydrosoluble réticulé peut être obtenu par un procédé comprenant :
(a) la polymérisation de substantiellement la totalité d'un halogénure de diallyldialkylammonium par une réaction initiée par un initiateur radicalaire pour former une solution de polymère cationique de base ;
(b) la mise en contact de la solution de polymère cationique de base avec un initiateur radicalaire supplémentaire pour former des liaisons d'interconnexion entre plusieurs groupements polymères cationiques, de sorte que ladite solution polymère cationique de base forme une solution aqueuse contenant un polymère cationique poly-réticulé.

2. Procédé conforme à la revendication 1, où la formule élaborée comprend entre 0,05 et 20 % en masse d'au moins un polymère cationique hydrosoluble réticulé de masse moléculaire moyenne en poids supérieure à 700 000 g/mol conformément à la revendication 1 et au moins un vecteur ou diluant de qualité cosmétique différent de l'eau ou en plus de l'eau.

3. Procédé conforme à la revendication 1, où au moins un halogénure de diallyldialkylammonium monomère est représenté par la formule (1) : 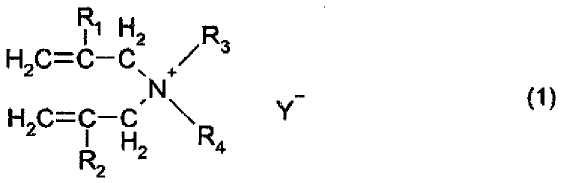 où
R₁ et R₂ représentent indépendamment l'un de l'autre un atome d'hydrogène ou un groupement alkyle en C₁-C₄ ;
R₃ et R₄ représentent indépendamment l'un de l'autre un atome d'hydrogène ou un groupement alkyle, hydroxyalkyle, carboxyalkyle, carboxyamidalkyle ou alkoxyalkyle comportant entre 1 et 18 atomes de carbone ;
et Y⁻ représente un anion.

4. Procédé conforme à la revendication 3, où le ou les halogénures de diallyldiméthylammonium monomère sont le chlorure de diallyldiméthylammonium, le bromure de diallyldiméthylammonium, le sulfate de diallyldiméthylammonium, le phosphate de diallyldiméthylammonium, le chlorure de diméthyallyldiméthylammonium, le chlorure de diéthylallyldiméthylammonium, le chlorure de diallyldi(bêta-hydroxyéthyl)ammonium, le chlorure de diallyldi(bêta-éthoxyéthyl)ammonium ou le chlorure de diallyldiéthylammonium,

5. Procédé conforme à la revendication 1, où le polymère cationique hydrosoluble réticulé présente une architecture moléculaire légèrement réticulée et/ou ramifiée et comprend un polymère de masse moléculaire élevée de chlorure de diallyldiméthylammonium pouvant être obtenu par réticulation post-réactionnelle.

6. Méthode non thérapeutique de traitement capillaire qui comprend la mise en contact des cheveux avec une formule de conditionnement capillaire, comprenant
a) 0,05 à 10 % en masse, par rapport à la masse totale de la formule, d'au moins un polymère cationique hydrosoluble réticulé conforme à la revendication 1,
b) 0,5 à 5 % en masse, par rapport à la masse totale de la formule, d'au moins un alcool gras à longue chaîne,
c) au moins un acide compatible avec la peau à une teneur suffisante pour obtenir un pH compris entre 2,5 et 5,5,
d) 0 à 5 % en masse, par rapport à la masse totale de la formule, d'au moins un adjuvant supplémentaire, et
e) de l'eau jusqu'à 100 % en masse.

7. Méthode conforme à la revendication 6 qui comprend la mise en contact des cheveux avec une formule de conditionnement capillaire sous forme de produits de rinçage à appliquer après le shampooing, avant ou après la teinture, la coloration ou la décoloration, et avant ou après un frisage ou un défrisage permanent ; les produits fixants ou coiffants ; les compositions de conditionnement ; les compositions de régénération ; ou les compositions pour cheveux à frisage permanent.

8. Méthode conforme à la revendication 7, qui comprend la mise en contact des cheveux avec une formule de soins capillaires qui est appliquée avant, pendant ou après une étape de défrisage.
